# EUROPÄISCHE PATENTSCHRIFT

(11) **EP 3 369 397 B1**
(45) Veröffentlichungstag und Bekanntmachung des Hinweises auf die Patenterteilung: **04.11.2020**
(21) Anmeldenummer: 18159192.6
(22) Anmeldetag: 28.02.2018
(51) Int. Cl.: A61D 19/04

(54) **ANORDNUNG ZUR ULTRASCHALLGESTÜTZTEN MANIPULATION AN DEN WEIBLICHEN FORTPFLANZUNGSORGANEN GROSSER SÄUGETIERE**
ASSEMBLY FOR ULTRASOUND-ASSISTED MANIPULATION AT THE FEMALE REPRODUCTIVE ORGANS OF LARGE MAMMALS
DISPOSITIF DE MANIPULATION PAR ULTRASONS D'ORGANES DE REPRODUCTION FEMELLES DES GRANDS MAMMIFÈRES

(30) Priorität: 01.03.2017 DE 102017002614
(43) Veröffentlichungstag der Anmeldung: 05.09.2018
(73) Patentinhaber: Hildebrandt, Thomas, 12557 Berlin (DE); Schnorrenberg, Arno, 15569 Woltersdorf (DE)
(72) Erfinder: HILDEBRANDT, Thomas, 12557 Berlin (DE); SCHNORRENBERG, Arno, 15569 Woltersdorf (DE); WEIßMANN, Ivo, 71034 Böblingen (DE)
(74) Vertreter: Müller, Wolfram Hubertus

(56) Entgegenhaltungen:
- EP-A2- 1 967 147
- WO-A2-2011/145818
- JP-A- H11 290 358

## Beschreibung

Die Erfindung betrifft eine Anordnung zur ultraschallgestützten Manipulation an den weiblichen Fortpflanzungsorganen großer Säugetiere, wie Nashorn, Elefant und Flusspferd. Die Erfindung ist insbesondere darauf gerichtet, die Reproduktion vom Aussterben bedrohter Tierarten zu unterstützen.

Für Oozytengewinnung am lebenden Tier ist die sogenannte Ovum Pick-Up Methode bekannt. Diese Methode basiert auf der Ultrasonographie und wird bisweilen auch als "transvaginale ultraschallgeleitete Follikelpunktion" bezeichnet. Bei ihr wird der Schallkopf in die Vagina geschoben und das Ovar durch die rektal eingeführte Hand gegen die Sonde gepresst. Meist durch eine zweite Person wird mit einer im Sondenträger angeordneten Kanüle der Follikel punktiert und die Eizelle entnommen.

Eine für die Gewinnung von Oozyten bei Kühen oder Pferden vorgesehene Vorrichtung und ein Verfahren dazu gehen aus der WO 2011145818 A2 hervor.

Die Vorrichtung umfasst einen Griff mit einem horizontal angeordneten Führungsrohr in dem eine Metallkanüle gelagert ist, die in axialer Richtung an ihrem vorderen Ende einen Ultraschallkopf aufweist der wiederum mit einer am oberen Ende des Griffes vorgesehenen Bildwiedergabeeinrichtung verbunden ist.

An ihrem vorderen Ende ist die Metallkanüle von einem vorwärts- und rückwärts beweglich gelagerten Schneidmesser umgeben, das zum Durchtrennen der Vaginalwand vorgesehen ist. Ein Oozyten-Sammelrohr nimmt die mittels einer Nadel gewonnenen Oozyten auf, die dann über einen Katheter zu einem Oozyten-Sammelbehälter durch Anlegen eines Vakuums transportiert werden.

Die Handhabung diese Vorrichtung gestaltet sich jedoch kompliziert. Nachdem der Bediener eine Hand durch das Rektum des Tieres gelegt hat, um den Uterus des Tieres zu halten, muss er die Metallkanüle mittels des Griffes und Führungsrohres durch den vaginalen Abschnitt des Tieres führen, um schließlich mit der Entnahme der Oozyten beginnen zu können.

Diese technische Lösung ist jedoch für die Entnahme von Oozyten bei Großtieren nicht geeignet. Eine transvaginale Follikelmethode scheidet bei Großtieren schon auf Grund der gegenüber Pferden und Kühen "riesigen" anatomischen Verhältnisse aus. So liegen zum Beispiel bei einem Nashorn die Eierstöcke ungefähr 1,5 m im Körperinneren.

Aus DE 102 03 094 B4 geht ein Besteck zur Gebärmutterhalspassage bei großen Säugetieren hervor. Dieses Besteck ist zwar für die Besamung derartiger Tiere geeignet nicht jedoch für die Oozytengewinnung und auch nicht für den Embryotransfer. Während der direkt nach dem Eisprung beginnenden zyklischen Gelbkörperphase verklebt ein sich bildender Schleim den zickzackförmigen Gebärmutterhalskanal und verhindert so das erfolgreiche Einbringen eines Katheters. Ein gewaltsames Öffnen dieser Passage würde das Tier verletzen und eine lokale medikamentöse Behandlung zu einer tödlichen Beschädigung der Fruchtanlage führen. Insofern schließt sich diese Möglichkeit der Gebärmutterhalspassage für den Embryotransfer von vornherein aus.

Aus der EP 1 967 147 A2 ist eine Punktionsvorrichtung für die Entnahme einer organischen Probe bekannt. Die Punktionsvorrichtung umfasst eine zu einer Nadelspitze zulaufende Punktionskanüle, deren proximales Ende mit einem Aufnahmebehälter für die organische Probe verbindbar ist, sowie eine Spülleitung, die entlang eines Abschnitts die Punktionskanüle umgibt und an einer Verbindungsstelle flüssigkeitdicht an dieser befestigt ist. Ein Führungsadapter dient der Verbindung der Punktionsvorrichtung mit einem Ultraschallkopf. Dabei ist eine keilförmige Auflage vorgesehen, die bewirkt, dass die Punktionskanüle in einen Winkel zwischen 3° und 10° zur Längsachse des Ultraschallkopfes verläuft.

Die JP H11 290358 A beschreibt einen Intra-Hohlraum-Manipulator mit einem Endoskop für die Entnahme einer Mehrzahl unbefruchteter Eizellen. Der Intra-Hohlraum-Manipulator wird in die Vagina eines Tieres eingeführt. Er umfasst ein Endoskop, das in ein zylindrisches Rohr eingesetzt wird.

Aufgabe der Erfindung ist es, eine Anordnung zur ultraschallgestützten Manipulation an weiblichen Fortpflanzungsorganen großer Säugetiere bereitzustellen, die diese Manipulation ohne Benutzung des vaginalen Abschnittes des Tieres ermöglicht und für die kontrollierte Entnahme von Oozyten sowie den Embryotransfer und die intrauterine Besamung unter Umgehung der schwierigen Gebärmutterkanal-Passage verwendet werden kann.

Erfindungsgemäß wird die Aufgabe zur ultraschallgestützten Manipulation an weiblichen Fortpflanzungsorganen großer Säugetiere durch eine Anordnung mit den Merkmalen des Anspruches 1 gelöst. Ausgestaltungen der Erfindung sind in den abhängigen Ansprüchen angegeben.

Danach stellt die Erfindung gemäß einem ersten Erfindungsaspekt eine Anordnung zur ultraschallgestützten Manipulation an weiblichen Fortpflanzungsorganen lebender großer Säugetiere wie Nashorn, Elefant und Flusspferd zur Verfügung. Diese umfasst ein Trägerrohr mit einem Abschlusselement und einem Griffstück, ein im Trägerrohr vorgesehenes Führungsrohr, eine Punktionsnadel, und einen am distalen Ende des Trägerohres vorgesehenen Ultraschallkopfträger mit Aufnahmen für einen Ultraschallkopf und des distalen Endes des Führungsrohres. Dabei ist die Achse des Ultraschallkopfes und des distalen Endes des Führungsrohres jeweils in Bezug auf die horizontale Achse des Trägerrohres so abgewinkelt, dass die Position der Spitze der Punktionsnadel im Ultraschall-Scanbereich des Ultraschallkopfes liegt. Der Ultraschallkopf ist mit einer Bildwiedergabeeinrichtung verbindbar bzw. verbunden.

Gemäß einer ersten Variante ist das Führungsrohr im Trägerrohr mittels eines passförmigen Positionierungselementes positioniert, die Punktionsnadel für einen Spül- und Absaugvorgang doppellumig ausgebildet, im Führungsrohr bewegbar angeordnet, mit ihrem proximalen Ende lösbar mit einem Impulsgeber verbunden und in diesem fixiert, wobei der Impulsgeber in dem Abschlusselement in horizontaler Richtung bewegbar gelagert ist, und am proximalen Ende der doppellumigen Punktionsnadel für die äußere und innere Nadel Anschlussstutzen vorhanden sind, die mittels Schläuchen mit einer medizinischen Spülvorrichtung und mit einer Vakuumpumpe und einem Aufnahmebehälter verbindbar sind. Diese erste Variante ist insbesondere zur rektalen Oozytengewinnung bei großen Säugetieren, wie Nashorn, Elefant und Flusspferd geeignet.

Gemäß einer zweiten Variante weist das Führungsrohr annähernd die Länge des Trägerrohres auf und ist dieses an seinem proximalen Ende im Abschlusselement gelagert. Innerhalb des Führungsrohres ist eine Einführungshilfe vorgesehen, in der eine einlumig ausgebildete Punktionsnadel horizontal bewegbar angeordnet ist. In der einlumigen Punktionsnadel ist ein bewegbarer Katheter vorgesehen, der an seinem proximalen Ende mit einer Luer-Lock-Verbindung versehen ist. Diese zweite Variante ist insbesondere für den rektalen Embryotransfer und die intrauterine Besamung von großen Säugetieren, wie Nashorn, Elefant und Flusspferd geeignet.

Mit der Erfindung sind erstmals die Voraussetzungen geschaffen worden, um bei großen Säugetieren auf rektalem Weg Oozyten entnehmen, Embryonen transferieren sowie die die intrauterine Besamung dieser Tiere unter Umgehung der schwierigen Gebärmutterkanal-Passage durchführen zu können. Die Erfindung erlaubt eine sichere und erfolgreiche Eizellgewinnung bei diesen Tiergruppen, bei denen die vaginale Eizellgewinnung aufgrund der anatomischen Verhältnisse ausgeschlossen ist. Die Erfindung ist insbesondere darauf gerichtet, die Reproduktion vom Aussterben bedrohter Tierarten zu unterstützen. Mit der erfindungsgemäßen Anordnung können ansonsten unfruchtbare weibliche Individuen von hochbedrohten Tierarten noch in die Vermehrung über den Umweg der In-Vitro-Fertilisation (IVF), auch Reagenzglas-Befruchtung genannt, mit einbezogen werden.

Gemäß einer Ausgestaltung der Erfindung ist bei der Anordnung die Achse des Ultraschallkopfes im Winkel von 30° bis 90° zur horizontalen Achse des Trägerrohres vorgesehen.

Bevorzugt weist ein Positionierungselement für das Führungsrohr eine trichterförmige Aufnahme zur Unterstützung des Einführens einer Punktionsnadel in das Führungsrohr auf.

Eine weitere Ausführungsform der Erfindung sieht vor, dass der Ultraschallkopfträger lösbar mit dem Trägerrohr verbunden ist.

Typischerweise ist die doppellumige Punktionsnadel zur Erhöhung der Gleitfähigkeit im Führungsrohr mit einem Teflonschlauch umgeben und/oder weist das Führungsrohr an seinem distalen Ende eine aufschraubbare Abschlusskappe auf.

Gemäß einer besonders bevorzugten Ausführungsform der Erfindung ist ein Impulsgeber an seinem distalen Ende aufgeschlitzt und wird in diesem Schlitz der Schlauch für die Spülflüssigkeit sowie der Schlauch für abgesaugte Oozyten anfangs geführt und werden danach beide Schläuche durch eine im Abschlusselement des Trägerrohres vorgesehene Bohrung geleitet.

Typischerweise wird die lösbare Verbindung zwischen dem proximalen Ende der Punktionsnadel und dem Impulsgeber mittels einer auf den Impulsgeber aufschraubbaren Überwurfhülse hergestellt.

Eine weitere Ausführungsform der Erfindung sieht vor, dass zur Begrenzung der Wegstrecke der distanzgeführten Punktionsnadel am Impulsgeber innerhalb des Trägerrohres und außerhalb des Trägerrohres jeweils ein Anschlagelement vorgesehen ist.

Typischerweise ist am Impulsgeber ein Arretierelement mit Feststellschraube zur Fixierung der passiven Funktion der Vorrichtung und für die Freigabe zur aktiven Funktion vorgesehen.

Typischerweise sind Führungsrohre und Einführhilfe zweiteilig ausgebildet, sind aus Edelstahl gefertigt und können lösbar miteinander verbunden werden.

Vorzugsweise können die Führungsrohre zur Erhöhung deren Gleitfähigkeit mit einem dafür geeigneten Kunststoff überzogen werden.

Typischerweise sind die Punktionsnadel einlumig ausgebildet und der Katheter so gelagert, dass sie von Hand führbar sind und ist innerhalb des Katheters eine Einwegspritze oder eine Mikropipette vorgesehen.

Ausgestaltungen der Vorrichtung zur Entnahme von Oozyten bei lebenden Tieren geben die Ansprüche 2 bis 12 an.

Weitere Aspekte dieser Offenbarung, die nicht Teil der Erfindung sind, betreffen die Verwendung einer erfindungsgemäßen Anordnung zur ultraschallgestützten Manipulation an weiblichen Fortpflanzungsorganen zur rektalen Oozytengewinnung bei großen Säugetieren, wie Nashorn, Elefant und Flusspferd.

Es ist das besondere Verdienst der Erfinder, dass mit der von ihnen gefundenen technischen Lösung erstmals die Voraussetzungen geschaffen wurden, um bei großen Säugetieren auf rektalem Weg Oozyten entnehmen und Embryonen transferieren sowie die die intrauterine Besamung unter Umgehung der schwierigen Gebärmutterkanal-Passage durchführen zu können.

Damit leisten die Erfinder einen sehr bedeutsamen Beitrag zum Artenschutz und zur Unterstützung der Reproduktion insbesondere von Großtieren, die vom Aussterben bedroht sind.

Mit der erfindungsgemäßen Anordnung können ansonsten unfruchtbare weibliche Individuen von hochbedrohten Tierarten noch in die Vermehrung über den Umweg der In-Vitro-Fertilisation (IVF) auch Reagenzglas-Befruchtung genannt mit einbezogen werden. 1978 kam das erste sogenannte Test Tube Baby Louise Brown in England zur Welt. Mit ihrer Geburt wurde eine neue Ära in der Fortpflanzungsmedizin beim Menschen eingeleitet. Die Nutztierindustrie folgte relativ schnell.

Im Artenschutz zur Rettung hochbedrohter Tierarten findet diese Technik bisher keine Anwendung, da insbesondere die anatomischen Verhältnisse bei Großtierarten, wie Nashorn, Elefant und Flusspferd, die direkte Übertragung der vaginalen Eizellaspiration nicht zulassen ohne den Patienten einem unkalkulierbaren Risiko auszusetzen. Die hier beschriebene Erfindung erlaubt eine sichere und erfolgreiche Eizellgewinnung bei diesen Tiergruppen, bei denen die vaginale Eizellgewinnung aufgrund der anatomischen Verhältnisse ausgeschlossen ist.

Beim Menschen werden jährlich mehrere Millionen Eizellentnahmen durchgeführt und es sind mehr als 7 Millionen Menschen unter Anwendung dieses Verfahrens geboren worden.

Die Erfinder sind davon überzeugt; dass mit ihrer neuen technischen Lösung dazu beigetragen wird, Arten, wie das nördliche Breitmaulnashorn oder das Sumatranashorn vor dem Aussterben zu bewahren. Ansonsten wären die unfruchtbaren Weibchen der bedrohten Tierarten für immer für die Zucht verloren.

Im Folgenden soll die Erfindung an Hand von Zeichnungen näher erläutert werden. Es zeigen:
- **Figur 1**: die Seitenansicht einer erfindungsgemäßen Vorrichtung zur Oozytengewinnung,
- **Figur 2**: die erfindungsgemäße Vorrichtung nach Figur 1 mit ihren wesentlichen Baugruppen,
- **Figur 3**: die Seitenansicht einer erfindungsgemäßen Vorrichtung zur Oozytengewinnung im Schnitt;
- **Figur 4**: den Impulsgeber in perspektivischer Darstellung,
- **Figur 5**: Seitenansicht des proximalen Abschnittes einer erfindungsgemäßen Vorrichtung zur Oozytengewinnung im Schnitt,
- **Figur 6**: in perspektivischer Darstellung den Abschnitt einer erfindungsgemäßen Vorrichtung mit geöffnetem Ultraschallkopfträger und eingelegter Punktionsnadel,
- **Figur 7**: in perspektivischer Darstellung den Abschnitt einer erfindungsgemäßen Vorrichtung mit integriertem Ultraschallkopf, und
- **Figur 8**: proximaler Abschnitt der erfindungsgemäßen Anordnung für den Embryotransfer und die intrauterine Besamung im Schnitt.

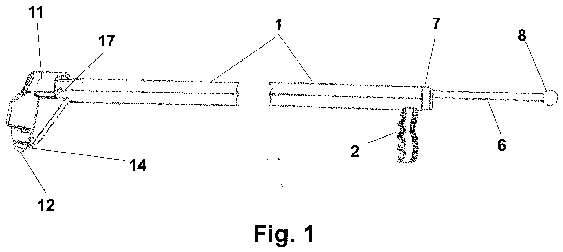

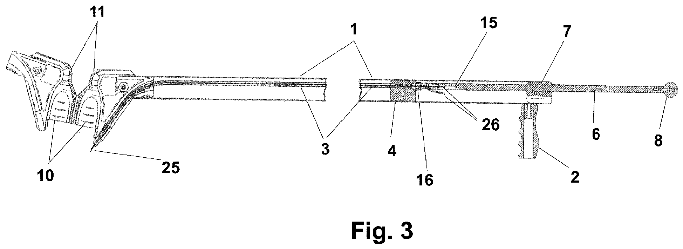

Wie aus **Fig. 1 bis Fig. 3** und **Fig. 5** zu erkennen weist die erfindungsgemäße Vorrichtung ein Trägerrohr 1 mit einem Griff 2 auf. An seinem proximalen Ende ist es mit einem Abschlusselement 7 versehen, durch das ein Impulsgeber 6 mit einem Griffelement 8 geführt wird. Am distalen Ende des Trägerrohres 1 ist ein Ultraschallkopfträger 11 mittels einer Schraube 17 lösbar mit dem Trägerrohr 1 verbunden. Eine Bohrung 22 ist für die Befestigung des Griffes 2 am Trägerrohr 1 vorgesehen. Die erfindungsgemäße Anordnung hat im vorliegenden Ausführungsbeispiel zur Verwendung für eine Oozytengewinnung bei einer Nashornkuh eine Gesamtlänge von 1400 mm. Je nach der Tierart kann die Gesamtlänger der Anordnung im Bereich zwischen 1300 mmm und 2000 mm liegen. Der äußere Durchmesser des Trägerrohres 1 liegt im vorliegenden Beispiel bei ca. 45 mm.

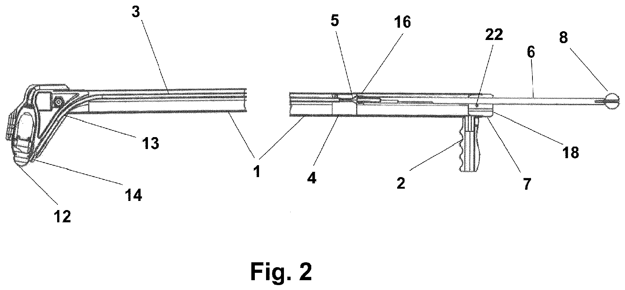

Das Trägerrohr 1 dient der Aufnahme des Führungsrohres 3 in der die doppellumige Punktionsnadel 25 mit einer Länge von ca. 900 mm gleitet. Das distale Ende 13 des Führungsrohres 3 mit der aufschraubbaren Abschlusskappe 14 ist, wie aus Fig. 2 zu sehen ist, abgewinkelt. Von besonderer Bedeutung ist, die Abwinklung des Führungsrohres 3 im Verhältnis zur Achse des Trägerrohres 1 so vorzusehen, dass für die Oozytenentnahme die Spitze der doppellumigen Punktionsnadel 25 im Scanfeld des Ultraschallkopfes 12 liegt. Da die Achse des Ultraschallkopfes 12 im Verhältnis zur horizontalen Achse des Trägerrohres 1 einen Winkel von 30° bis 90 ° einnehmen kann, ist eine präzise Fertigung des Ultraschallkopfträgers 11 mit den Aufnahmen 10 für den Ultraschallkopf 12 sowie mit den Aufnahmen für das Führungsrohr 3 und die Abschlusskappe 14 des Führungsrohres 3 unabdingbar.

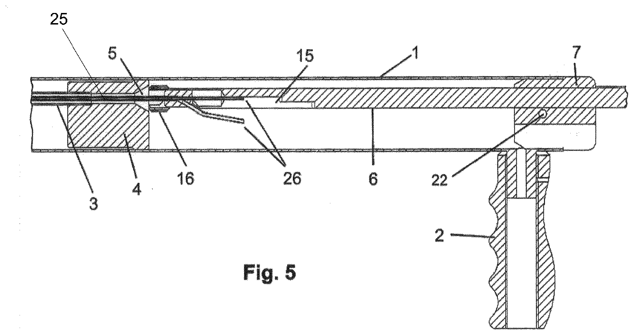

An seinem anderen Ende ist das Führungsrohr 3 in dem Positionierungselement 4 gelagert, das passförmig im Trägerrohr 1 angeordnet ist. Um das Einführen der doppellumigen Punktionsnadel 25 in das Führungsrohr 3 zu unterstützen weist das Positionierungselement 4, wie auch aus **Fig. 5** zu entnehmen ist, die trichterförmige Aufnahme 5 auf. Für eine bessere Gleitfähigkeit der Punktionsnadel 25 im Führungsrohr 3, insbesondere in dessen abgewinkelten distalen Ende 13, ist die Punktionsnadel mit einem Teflonschlauch umgeben. Es kann jedoch auch ein Schlauch aus einem anderen die Gleitfähigkeit unterstützenden Kunststoff dafür verwendet werden.

Hat die Spitze der Punktionsnadel 25 die Öffnung der Abschlusskappe 14 erreicht, wird das andere Ende der Punktionsnadel 25 mittels der aufschraubbaren Überwurfhülse 16 mit dem Impulsgeber 6 lösbar verbunden. Die Punktionsnadel 25 ist damit zugleich im Impulsgeber 6 fixiert. Hier nicht dargestellt ist, dass die Anschlussstutzen 26 der inneren Hohlnadel und der äußeren Hohlnadel der doppellumigen Punktionsnadel 25 jeweils mit einem sterilen Schlauch verbunden werden. Die Schläuche werden zunächst in dem geschlitzten Ende 15 des Impulsgebers 6 geführt und anschließend durch die Bohrung 18 im Abschlusselement 7 aus der Anordnung abgeleitet.

Je nachdem welche der Hohlnadeln für den Spülvorgang oder für das Absaugen der Oozyten genutzt wird, ist diese mit einer hier nicht dargestellten medizinischen Spülvorrichtung oder einer Vakuumpumpe mit einem sterilen Auffang-Container für die Aufnahme der abgesaugten Oozyten verbunden. Zur Anwendung kommen doppellumige Punktionsnadeln mit einem Durchmesser von ca. 1,6 mm bis 2,5 mm für die äußere Hohlnadel und ca. 1,4 mm bis 1,8 mm für die innere Hohlnadel.

Die führbare Wegstrecke des Impulsgebers 6 wird durch hier nicht dargestellte am Impulsgeber 6 vorgesehene zwei Anschlagelemente begrenzt. Zwischen diesen zwei Anschlagelementen des Impulsgebers 6 befindet sich das Abschlusselement 7, womit sichergestellt ist, dass der Impulsgeber 6 nicht unbeabsichtigt aus dem Trägerrohr 1 entfernt werden kann. Die führbare Wegstrecke des Impulsgebers 6 kann zwischen 60 mm und 120 mm liegen. Mittels eines hier nicht dargestellten am Impulsgeber 6 außerhalb des Trägerrohres 1 vorgesehenen Arretierelementes mit Bajonettverschluss oder von Feststellschrauben kann die passive Funktion der Anordnung fixiert oder die aktive Funktion des Impulsgebers 6 wieder freigegeben werden.

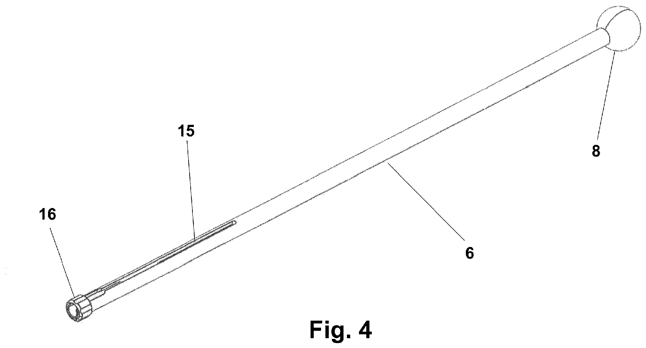

Die **Fig. 4** zeigt in perspektivischer Darstellung den Impulsgeber 6, der aus Edelstahl gefertigt ist. Deutlich zu erkennen ist die aufschraubbare Überwurfhülse 16 um den Impulsgeber 6 mit der doppellumigen Punktionsnadel 25 verbinden zu können. Der Schlitz 15 dient - wie schon beschrieben - der Durchführung der an die Anschlussstutzen 26 anzuschließenden sterilen Schläuche.

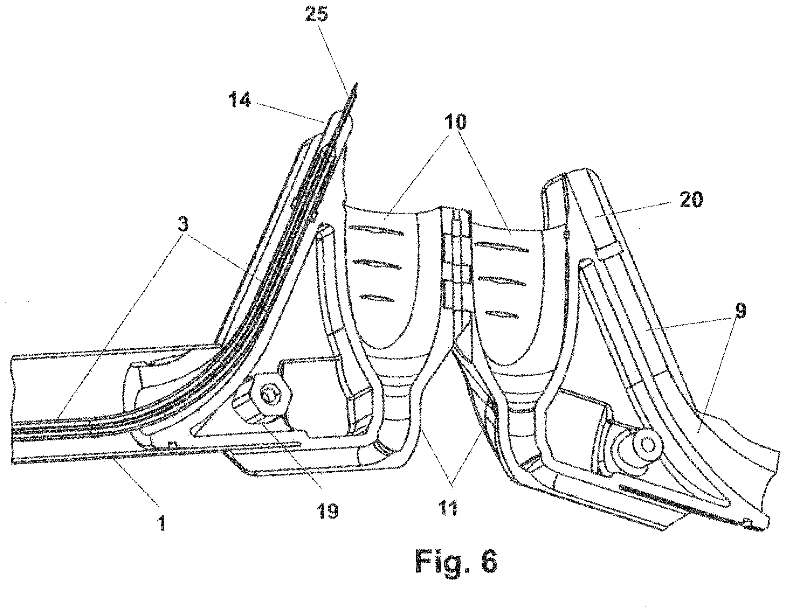

**Fig. 6** zeigt in perspektivischer Darstellung den Ultraschallkopfträger 11 in geöffneter Darstellung mit der doppellumigen Punktionsnadel 25. Deutlich dargestellt sind die Aufnahme 20 für die Abschlusskappe 14 sowie die Aufnahme 9 für das Führungsrohr 3. Es hat sich bewährt, dieses Element der erfindungsgemäßen Anordnung mittels eines 3D-Druckers aus einem hochfesten sterilisierbaren Kunststoff zu fertigen.

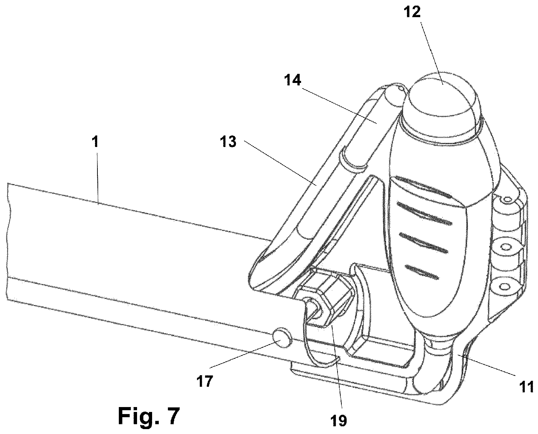

Aus **Fig. 7** geht in perspektivischer Darstellung das Endstück der erfindungsgemäßen Anordnung mit dem Ultraschallkopfträger 11 und dem darin integrierten Ultraschallkopf 12 sowie das distale Ende 13 des Führungsrohres 3 mit der aufgeschraubten Abschlusskappe 14 hervor. Mittels der Schraubverbindung 17, 19 ist der Ultraschallkopfträger 11 im Trägerrohr 1 verankert und positioniert. Der Ultraschallkopf 12 ist mit einem hier nicht dargestellten Bildwiedergabesystem verbunden. Die Verbindungsleitung wird entlang des Trägerrohres 1 geführt.

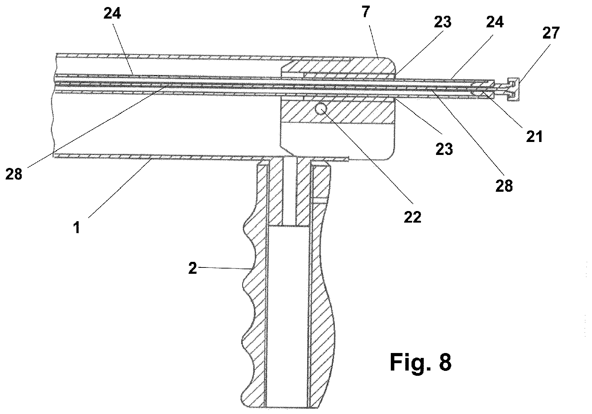

Im Schnitt ist in **Fig. 8** das proximale Ende einer für den Embryotransfer und die intrauterine Besamung vorgesehenen erfindungsgemäßen Anordnung dargestellt. Eine solche Anordnung verfügt über das Führungsrohr 24 und die Einführhilfe 28 für die einlumige Punktionsnadel. Das Führungsrohr 24 und die aus Edelstahl gefertigte Einführhilfe 28 reichen vom Ultraschallkopfträger 11 bis ca. 30 mm über das Ende des Abschlusselementes 7 hinaus.

Um zu gewährleisten, dass das Trägerrohr 1 und das Abschlusselement 7 in der Anordnung bevorzugt sowohl zur Oozytengewinnung als auch für den Embryotransfer und die intrauterine Besamung verwendet werden können, ist die Hülse 23 für die sichere Positionierung des Führungsrohres 24 vorgesehen. Das Fixierelement 21 gewährleistet eine sichere Positionierung der Einführhilfe 28 die innerhalb des Führungsrohres 24 angeordnet ist.

Am Ende der Einführhilfe 28 ist das aufschraubbare Distanzelement 27 vorgesehen damit die Einführhilfe 28 manipuliert werden kann bis deren distales Ende ca. 2 mm aus der Abschlusskappe 14 hinausragt.

Für den Embryotransfer und die intrauterine Besamung werden in die Einführhilfe 28 die hier nicht dargestellte einlumige Punktionsnadel mit einer Länge von ca. 1200 mm bis 2000 mm und in diese ein ebenfalls hier nicht dargestellter Katheter aus Plastik mit einer Länge von ca. 1300 mm bis 2100 mm eingeführt.

Die Punktionsnadel mit einem Außendurchmesser von ca. 1,5 mm und einem Innendurchmesser von ca. 1,1 mm und der Katheter mit einem Außendurchmesser von ca. 1,0 mm und einem Innendurchmesser von ca. 0,5 mm, sind jeweils per Hand manipulierbar.

Im Folgenden soll die Verwendung der Anordnung zur Oozytengewinnung sowie zum Embyotransfer und zur intrauterinen Besamung an einem Nashornweibchen beschrieben werden.

Das weibliche Nashorn (es gibt 6 Arten und für alle 6 Arten kann die Prozedur angewandt werden) wird für den jeweiligen Eingriff entweder hormonell stimuliert oder es erfolgt die Gewinnung der Eizellen ohne Stimulierung der Eierstöcke. Der Embryotransfer erfolgt am Tag 2 bis 10 nach der Ovulation mit dem Vorhandensein eines Gelbkörpers am Eierstock. Die intrauterine Besamung wird zum Zeitpunkt des erwarteten Eisprungs bzw. direkt danach durchgeführt. Die Nicht-Stimulierung hat zur Folge, dass die Gesamtausbeute an Eizellen gesenkt sein kann, aber die Prozedur kann in kürzeren Abständen wiederholt werden.

Das Nashorn wird für den Eingriff in Vollnarkose versetzt und während der gesamten Prozedur von einem Anästhesisten-Team betreut.

Wenn das Tier in Brustbein- oder Seitenlage im Stall zum Liegen kommt, wird zuerst der Kot im Enddarm auf einer Länge von ca. 2 m manuell entfernt und danach die Darmschleimhaut von den noch anheftenden Kotpartikeln mittels intensiver Spülung mit Trinkwasser über ein Schlauchsystem (Zulauf-Wasserhahn, Ablauf-spontan durch Gefälle) gereinigt. Danach erfolgt die fraktionierte Einbringung eines kommerziell erhältlichen medizinischen Schleimhautdesinfektionsmittels. Es werden jeweils in 5 Liter-Schritten die Desinfektionslösung (Stammlösung laut Anweisung mit Trinkwasser verdünnt um Gebrauchslösung herzustellen) in den mechanisch gereinigten Darmabschnitt eingebracht und dann wieder mit einem Ablaufschlauchsystem vollständig entfernt. Diese Vorgehensweise wird 3-mal wiederholt. Damit ist der Enddarm mechanisch gereinigt und desinfiziert.

Parallel zu diesen Arbeiten am Nashorn werden die jeweiligen modularen Anordnungen zur Nashorn-Oozytengewinnung, dem Embryotransfer oder der intrauterinen Besamung für den Eingriff - wie mit **Fig. 1** bis **Fig. 8** dargestellt und beschrieben - vorbereitet. Zur Oozytengewinnung gehören die Zusatzkomponenten des Systems, bestehend aus der medizinischen Vakuumpumpe (Fa. Cook GmbH), dem sterilen Auffangbehälter (50 ml Röhrchen mit Schraubverschluss, kommerziell Fa. Greiner GmbH) und der 60 ml Luer-Lock-Spritze gefüllt mit kommerzieller Follikel-Spülflüssigkeit (Produktname Euroflush). Die 60 ml Spritze mit der Spülflüssigkeit ist in ein mechanisches Dosierungssystem (Fa. Steiner, Graz Österreich) mit integrierter Wärmefunktion (37°C) und Nachfülleinrichtung eingesetzt und wird über ein Fußpedal bedient. Die Einzelkomponenten werden mit Hilfe eines sterilen Schlauchsystems einschließlich Silikonstopfen mit integriertem Vakuumanschluss (Fa. Gynetics Belgien) verbunden.

Für den Embryotransfer bzw. für die intrauterine Besamung wird die geringfügig modifizierte Anordnung zur Oozytengewinnung eingesetzt. Der Grundaufbau der Anordnung für den Embryotransfer unterscheidet sich nur marginal vom Aufbau der Anordnung zur Oozytengewinnung, wobei die Komponenten wie Vakuumpumpe, steriler Auffangbehälter oder die Spülflüssigkeit nicht erforderlich sind. Die doppelumige Nadel wird für den Transfer bzw. für die Besamung durch eine längere einlumige Nadel ersetzt.

Für das problemfreie Einführen der erfindungsgemäßen Anordnung in den gereinigten und desinfizierten Enddarm des Nashorns wird der vordere Bereich (Schallkopf 12, Schallkopfhalterung 11 und ca. 20 cm des Hüllrohrs 1) mit einer dünnen Schicht von sterilem medizinischem Gleitgel (PreSeed, USA) vollständig überzogen. Die Anordnung wird, abhängig von der Größe des Tieres, ca. 1 m bis 1,7 m in den Enddarm eingeführt. Der am distalen Ende angeordnete Ultraschallkopf 12 wird im vorliegenden Fall mit dem portablen Ultraschallsystem (Voluson i, Fa. GE HealthCare GmbH) verbunden.

Für die Oozytengewinnung wird jeweils der rechte und linke Eierstock aufgesucht. Die integrierte Biopsie-Funktion des Ultraschallsystems (angezeigte Punktionslinie mit Längenangaben) erlaubt die genaue Planung der Follikelaspiration und -spülung.

An jedem Eierstock werden dann systematisch alle Follikel mit einer Größe über 5 mm Durchmesser aspiriert und jeweils bis zu 10 Mal der aspirierte Follikel wieder zur Ursprungsgröße aufgefüllt (Spülung) und anschließend wieder aspiriert. Dieses Vorgehen erhöht deutlich die Chancen, dass sich die Eizelle in der Spülflüssigkeit befindet. Das Einstechen der doppellumigen Punktionsnadel 25 in die am Eierstock befindlichen Follikel erfolgt jeweils durch die Darmwand, es sei denn Follikel liegen nebeneinander- oder untereinander. In einem solchen Fall werden sie mit dem gleichen Zugang aspiriert. Die doppellumige Nadel 25 wird jeweils zurück in das Trägerrohr 1 gezogen (innerer Anschlag) und die Position für die nächste Punktion mittels des Ultraschallbildes optimal eingestellt.

Die während einer Follikelpunktion abgesaugte Flüssigkeit wird steril in dem Auffang-Container (Volumen 50 ml, steht in einem Wärmesystem der Berliner Firma Butter Ingenieurbüro, Deutschland) gewonnen, wobei die Wechselung jeweils nach Erreichen der Füllmarke von 40 ml erfolgt, um ein Einsaugen der Aspirationsflüssigkeit in das mit der Vakuumpumpe verbundenen Schlauchsystems zu vermeiden.

Nach erfolgreicher Follikelaspiration und -spülung wird die Anordnung zur Oozytengewinnung aus dem Enddarm entfernt.

Die Vorgehensweisen beim Embryotransfer bzw. bei der intrauterinen Besamung sind identisch.

Das distale Ende der Anordnung wird mittels Ultraschallkontrolle so über dem Uterushorn positioniert, dass die in die Einführhilfe 28 eingebrachte einlumige Nadel per Hand unter sonographischer Sichtkontrolle durch die Darmwand und die Mitte des Gebärmutterhorns eingestochen werden kann. Es wird jenes Gebärmutterhorn gewählt, an dessen Ende sich der Eierstock mit dem Graafschen Follikel bzw. dem frischen Gelbkörper befindet.

Die Nadelspitze wird exakt im Inneren der Gebärmutterhöhle positioniert, um dann einen flexiblen Katheter mit einer Länge von ca. 1300 mm bis 2100 mmm und einem äußeren Durchmesser von ca. 1,0 mm durch das innere Lumen der positionierten einlumigen Nadel bis in die Gebärmutterhöhle per Hand schieben zu können. Der Katheter aus einem bioverträglichen Material wird mit Hilfe einer an der Punktionsnadel angebrachten trichterförmigen Einführhilfe ohne Hängenbleiben in die Punktionsnadel eingeführt. Über diesen flexiblen Katheter wird dann entweder der zu transferierende Embryo oder das Sperma in die Gebärmutterhöhle verbracht.

Die Spitze des Katheters ist gerundet und sein Ende ist mit einer sogenannten kommerziell erhältlichen Quetsch-Luer-Lock-Verbindung verbunden. Damit kann bei einer Besamung das Sperma, gefüllt in einer 2 ml bis 5 ml sterilen Einwegspritze, über den Katheter in das Gebärmutterlumen verbracht werden.

Für den Embryotransfer wird der Katheter ebenfalls manuell mehrere Zentimeter über die Nadelspitze der einlumigen Nadel hinaus mit der Hand unter sonographischer Sichtkontrolle in Richtung Gebärmutterhornspitze geschoben.

Die Spitze des Katheters wird mehrere Zentimeter in das Gebärmutterlumen vorgeschoben, um etwas Abstand zur Nadel und zu möglichem vom Einstich stammenden Blut zu bekommen.

Nach optimaler Positionierung der Katheterspitze erfolgt die Entleerung des sich im Katheter befindlichen Embryo über den Anschluss einer kommerziellen Mikropipette (20 microliter Hub) mit der Luer-Lock-Quetschverbindung. Beim Pressen des Stempels der Mikropipette wird der sich in einer ca. 1 mm langen Flüssigkeitssäule befindliche Embryo schonend in die Gebärmutter entlassen.

Da das Sperma ein deutlich größeres Volumen ausmacht (2 ml bis 5 ml) wird dafür eine Einwegspritze verwendet. Der Embryo wird nur in eine ganz kleine Flüssigkeitssäule eingesogen und dann von hinten mittels der Micropipette über eine Luftsäule ausgedrückt.

Nach erfolgtem Embryotransfer bzw. erfolgter Besamung werden Katheter und einlumige Nadel nacheinander aus dem Tier entfernt.

Anschließend können noch topisch wirkende Antibiotika-Schaumstifte (Produkt aus der Rinderproduktion) in den Darm eingebracht werden, um möglichen lokalen Entzündungen vorzubeugen.

Die jeweils verwendete Anordnung wird anschließend auseinandergebaut, grob gereinigt und desinfiziert. Die einzelnen Bauelemente werden danach sterilisiert.

Im Anschluss der direkten Eizellgewinnung am Tier werden die Eizellen mit Hilfe eines Stereomikroskops (Fa. Carl Zeiss Jena GmbH) in der Spülflüssigkeit aufgesucht, in eine Waschlösung mittels Mikropipette überführt und danach für den Versand in 2 ml Container mit Hälterungsmedium verbracht.

### BEZUGSZEICHENAUFSTELLUNG

- 1: Trägerrohr
- 2: Griffstück
- 3: Führungsrohr
- 4: Positionierungselement
- 5: trichterförmige Aufnahme
- 6: Impulsgeber
- 7: Abschlusselement
- 8: Griffelement
- 9: Aufnahme für Führungsrohr
- 10: Aufnahme für Ultraschallkopf
- 11: Ultraschallkopfträger
- 12: Ultraschallkopf
- 13: distales Ende
- 14: Abschlusskappe
- 15: Schlitz
- 16: aufschraubbare Überwurfhülse
- 17: Schraube
- 18: Bohrung
- 19: Schraubverbindung
- 20: Aufnahme für Abschlusskappe
- 21: Fixierelement
- 22: Bohrung
- 23: Hülse
- 24: Führungsrohr
- 25: Punktionsnadel
- 26: Anschlussstutzen
- 27: Distanzelement
- 28: Einführhilfe für Katheter

## Patentansprüche

1. Anordnung zur ultraschallgestützten Manipulation an weiblichen Fortpflanzungsorganen lebender großer Säugetiere, wobei die Anordnung dazu vorgesehen und ausgebildet ist, für eine rektale Oozytengewinnung oder einen rektalen Embryotransfer oder eine rektale intrauterine Besamung in den Enddarm des großen Säugetiers eingeführt zu werden,
wobei die Anordnung umfasst:
- ein Trägerrohr (1) mit einem Abschlusselement (7) und einem Griffstück (2),
- ein im Trägerrohr (1) vorgesehenes Führungsrohr (3; 24),
- eine Punktionsnadel (25),
- einen am distalen Ende (13) des Trägerohres (1) vorgesehenen Ultraschallkopfträger (11) mit Aufnahmen (10) für einen Ultraschallkopf (12) und des distalen Endes (13) des Führungsrohres (3; 24),
- wobei die Achse des Ultraschallkopfes (12) und des distalen Endes (13) des Führungsrohres (3; 24) jeweils in Bezug auf die horizontale Achse des Trägerrohres (1) so abgewinkelt sind, dass die Position der Spitze der Punktionsnadel (25) im Ultraschall-Scanbereich des Ultraschallkopfes (12) liegt, und
- sofern die Anordnung dazu vorgesehen und ausgebildet ist, für eine rektale Oozytengewinnung in den Enddarm des großen Säugetiers eingeführt zu werden:
- die Anordnung des Weiteren einen Impulsgeber (6) umfasst, der in dem Abschlusselement (7) in horizontaler Richtung bewegbar gelagert ist,
- das Führungsrohr (3) im Trägerrohr (1) mittels eines passförmigen Positionierungselementes (4) positioniert ist,
- die Punktionsnadel (25) für einen Spül- und Absaugvorgang doppellumig ausgebildet, im Führungsrohr (3) bewegbar angeordnet, mit ihrem proximalen Ende lösbar mit dem Impulsgeber (6) verbunden und in diesem fixiert ist, und
- am proximalen Ende der doppellumigen Punktionsnadel (25) für die äußere und innere Nadel Anschlussstutzen (26) vorhanden sind, die mittels Schläuchen mit einer medizinischen Spülvorrichtung und mit einer Vakuumpumpe und einem Aufnahmebehälter verbindbar sind,
oder
- sofern die Anordnung dazu vorgesehen und ausgebildet ist, für einen rektalen Embryotransfer oder eine rektale intrauterine Besamung in den Enddarm des großen Säugetiers eingeführt zu werden:
- das Führungsrohr (24) annähernd die Länge des Trägerrohres (1) aufweist und an seinem proximalen Ende im Abschlusselement (7) gelagert ist,
- innerhalb des Führungsrohres (24) eine Einführungshilfe (28) vorgesehen ist, in der eine einlumig ausgebildete Punktionsnadel horizontal bewegbar angeordnet ist und
- in der einlumigen Punktionsnadel ein bewegbarer Katheter vorgesehen ist, der an seinem proximalen Ende mit einer Luer-Lock-Verbindung versehen ist.

2. Anordnung nach Anspruch 1, **dadurch gekennzeichnet ist, dass** die Achse des Ultraschallkopfes (12) im Winkel von 30° bis 90° zur horizontalen Achse des Trägerrohres (1) angeordnet ist.

3. Anordnung nach Anspruch 1 oder 2, **dadurch gekennzeichnet, dass** das Positionierungselement (4) für das Führungsrohr (3) eine trichterförmige Aufnahme (5) zur Unterstützung des Einführens der doppellumigen Punktionsnadel in das Führungsrohr (3) aufweist.

4. Anordnung nach mindestens einem der Ansprüche 1 bis 3, **dadurch gekennzeichnet, dass** der Ultraschallkopfträger (10) lösbar mit dem Trägerrohr (1) verbunden ist.

5. Anordnung nach mindestens einem der Ansprüche 1 bis 4, **dadurch gekennzeichnet, dass** die doppellumige Punktionsnadel zur Erhöhung der Gleitfähigkeit im Führungsrohr (3) mit einem Teflonschlauch umgeben ist und/oder das Führungsrohr (3) an seinem distalen Ende (13) eine aufschraubbare Abschlusskappe (14) aufweist.

6. Anordnung nach mindestens einem der Ansprüche 1 bis 5, **dadurch gekennzeichnet, dass** der Impulsgeber (6) an seinem distalen Ende aufgeschlitzt ist und in diesem Schlitz (15) der Schlauch für die Spülflüssigkeit sowie der Schlauch für abgesaugte Oozyten anfangs geführt sind und dann beide Schläuche durch die Bohrung (18) des Abschlusselementes (7) des Trägerrohres (1) geleitet sind.

7. Anordnung nach mindestens einem der Ansprüche 1 bis 6, **dadurch gekennzeichnet, dass** die lösbare Verbindung zwischen dem proximalen Ende der doppellumigen Punktionsnadel und dem Impulsgeber (6) mittels einer auf den Impulsgeber (6) aufschraubbaren Überwurfhülse (16) hergestellt ist.

8. Anordnung nach mindestens einem der Ansprüche 1 bis 7, **dadurch gekennzeichnet, dass** zur Begrenzung der Wegstrecke der distanzgeführten doppellumigen Punktionsnadel am Impulsgeber (6) Anschlagelemente vorgesehen sind, jeweils ein Anschlagelement innerhalb und außerhalb des Trägerrohres (1).

9. Anordnung nach mindestens einem der Ansprüche 1 bis 8, **dadurch gekennzeichnet, dass** am Impulsgeber (6) ein Arretierelement mit Bajonettverschluss oder eine Feststellschraube zur Fixierung der passiven Funktion der Anordnung und für die Freigabe zur aktiven Funktion vorgesehen ist.

10. Anordnung nach Anspruch 1, **dadurch gekennzeichnet, dass** das Führungsrohr (24) und die Einführhilfe (28) zweiteilig ausgebildet und lösbar miteinander verbindbar sind.

11. Anordnung nach Anspruch 1 oder 10, **dadurch gekennzeichnet, dass** das Führungsrohr (24) zur Erhöhung der Gleitfähigkeit mit einem dafür geeigneten Kunststoff überzogen ist.

12. Anordnung nach mindestens einem der Ansprüche 1, 10 oder 11, **dadurch gekennzeichnet, dass** die einlumige Punktionsnadel und der Katheter von Hand führbar gelagert sind und/oder innerhalb des Katheters eine Einwegspritze oder eine Mikropipette vorgesehen ist.

13. Anordnung nach mindestens einem der vorangehenden Ansprüche, **dadurch gekennzeichnet, dass** die Anordnung dazu vorgesehen und ausgebildet ist, für eine rektale Oozytengewinnung oder einen rektalen Embryotransfer oder eine rektale intrauterine Besamung in den Enddarm eines Nashorns, Elefants oder Flusspferds eingesetzt zu werden.

## Claims

1. Assembly for ultrasound-assisted manipulation at female reproductive organs of living large mammals, wherein the assembly is intended and designed to be inserted into the rectum of the large mammal for rectal oocyte recovery or rectal embryo transfer or rectal intrauterine insemination,
wherein the assembly comprises:
- a support pipe (1) with an end element (7) and a handle piece (2),
- a guide pipe (3; 24) provided in the support pipe (1),
- a puncture needle (25),
- an ultrasound head support (11) provided at the distal end (13) of the support pipe (1) and having accommodators (10) for an ultrasound head (12) and the distal end (13) of the guide pipe (3; 24),
- wherein the axis of the ultrasound head (12) and of the distal end (13) of the guide pipe (3; 24) are in each case angled with respect to the horizontal axis of the support pipe (1) such that the position of the tip of the puncture needle (25) is in the ultrasound scan range of the ultrasound head (12), and
- if the assembly is intended and designed to be inserted into the rectum of the large mammal for rectal oocyte recovery:
- the assembly furthermore comprises a pulse generator (6) which is mounted in a movable manner in the end element (7) in the horizontal direction,
- the guide pipe (3) is positioned in the support pipe (1) by means of a fitted positioning element (4),
- the puncture needle (25) has a twin-lumen construction for a flushing process and aspiration process, is arranged in the guide pipe (3) in a movable manner, is connected via its proximal end to the pulse generator (6) in a detachable manner and is fixed in said pulse generator, and
- there are connecting pieces (26) at the proximal end of the twin-lumen puncture needle (25) for the outer and inner needles, which connecting pieces are connectable to a medical flushing device and to a vacuum pump and a receptacle by means of pieces of tubing,
or
- if the assembly is intended and designed to be inserted into the rectum of the large mammal for rectal embryo transfer or rectal intrauterine insemination:
- the guide pipe (24) has approximately the length of the support pipe (1) and is mounted at its proximal end in the end element (7),
- there is provided within the guide pipe (24) an insertion aid (28), in which a single-lumen puncture needle is arranged in a horizontally movable manner and
- there is provided in the single-lumen puncture needle a movable catheter, which is provided at its proximal end with a Luer-Lock connector.

2. Assembly according to Claim 1, **characterized in that** the axis of the ultrasound head (12) is arranged at an angle of from 30° to 90° in relation to the horizontal axis of the support pipe (1).

3. Assembly according to Claim 1 or 2, **characterized in that** the positioning element (4) for the guide pipe (3) has a funnel-shaped receiver (5) for assisting the insertion of the twin-lumen puncture needle into the guide pipe (3).

4. Assembly according to at least one of Claims 1 to 3, **characterized in that** the ultrasound head support (10) is connected to the support pipe (1) in a detachable manner.

5. Assembly according to at least one of Claims 1 to 4, **characterized in that** the twin-lumen puncture needle is surrounded by a piece of Teflon tubing in order to increase slidability in the guide pipe (3) and/or the guide pipe (3) has at its distal end (13) a screw-on end cap (14).

6. Assembly according to at least one of Claims 1 to 5, **characterized in that** the pulse generator (6) is slit at its distal end and the piece of tubing for the flush liquid and the piece of tubing for aspirated oocytes are initially guided in this slit (15) and both pieces of tubing are then guided through the hole (18) of the end element (7) of the support pipe (1).

7. Assembly according to at least one of Claims 1 to 6, **characterized in that** the detachable connection between the proximal end of the twin-lumen puncture needle and the pulse generator (6) is established by means of a sleeve retainer (16) which can be screwed onto the pulse generator (6).

8. Assembly according to at least one of Claims 1 to 7, **characterized in that** the route of the distance-guided twin-lumen puncture needle is limited by providing on the pulse generator (6) stop elements, a stop element within the support pipe (1) and a stop element outside the support pipe (1).

9. Assembly according to at least one of Claims 1 to 8, **characterized in that** there is provided on the pulse generator (6) a locking element with a bayonet lock or a locking screw for fixing the passive function of the assembly and for releasing the active function.

10. Assembly according to Claim 1, **characterized in that** the guide pipe (24) and the insertion aid (28) have a two-part construction and can be connected to one another in a detachable manner.

11. Assembly according to Claim 1 or 10, **characterized in that** the guide pipe (24) is coated with a suitable plastic in order to increase slidability.

12. Assembly according to at least one of Claims 1, 10 or 11, **characterized in that** the single-lumen puncture needle and the catheter are mounted such that they are guidable by hand and/or a disposable syringe or a micropipette is provided within the catheter.

13. Assembly according to at least one of the preceding claims, **characterized in that** the assembly is intended and designed to be inserted into the rectum of a rhinoceros, elephant or hippopotamus for rectal oocyte recovery or rectal embryo transfer or rectal intrauterine insemination.

## Revendications

1. Dispositif de manipulation, assistée par ultrasons, d'organes reproducteurs femelles de grands mammifères vivants, le dispositif étant prévu et conçu pour être introduit dans le rectum du grand mammifère afin de prélever des ovocytes par voie rectale ou de transférer un embryon par voie rectale ou d'effectuer une insémination intra-utérine par voie rectale,
le dispositif comprenant :
- un tube support (1) pourvu d'un élément de fermeture (7) et d'une pièce de préhension (2),
- un tube de guidage (3 ; 24) prévu dans le tube support (1),
- une aiguille de ponction (25),
- un support de tête à ultrasons (11) prévu à l'extrémité distale (13) du tube de support (1) et comportant des logements (10) destinés à une tête à ultrasons (12) et à l'extrémité distale (13) du tube de guidage (3 ; 24),
- l'axe de la tête à ultrasons (12) et celui de l'extrémité distale (13) du tube de guidage (3 ; 24) étant chacun inclinés par rapport à l'axe horizontal du tube support (1) de sorte que la position de la pointe de l'aiguille de ponction (25) est située dans la zone de balayage par ultrasons de la tête à ultrasons (12),
et
- si le dispositif est prévu et conçu pour être introduit dans le rectum du grand mammifère afin de prélever des ovocytes par voie rectale :
- le dispositif comprenant en outre un générateur d'impulsions (6) qui est monté dans l'élément de fermeture (7) de manière mobile dans la direction horizontale,
- le tube de guidage (3) étant positionné dans le tube support (1) au moyen d'un élément de positionnement ajusté (4),
- l'aiguille de ponction (25) étant conçue à double lumière pour un processus de rinçage et d'aspiration, étant disposée de manière mobile dans le tube de guidage (3), étant reliée par son extrémité proximale de manière amovible au générateur d'impulsions (6) et
étant fixée dans celui-ci, et
- des raccords (26) destinés aux aiguilles extérieure et intérieure étant situés à l'extrémité proximale de l'aiguille de ponction (25) à double lumière et pouvant être reliés au moyen de tuyaux à un dispositif de rinçage à usage médical et à une pompe à vide et à un récipient de collecte,
ou
- si le dispositif est prévu et conçu pour être introduit dans le rectum du grand mammifère afin de transférer un embryon par voie rectale ou d'effectuer une insémination intra-utérine par voie rectale :
- le tube de guidage (24) ayant approximativement la longueur du tube support (1) et étant monté à son extrémité proximale dans l'élément de fermeture (7),
- un auxiliaire d'introduction (28), dans lequel une aiguille de ponction à une seule lumière est disposée de manière mobile horizontalement, étant prévu à l'intérieur du tube de guidage (24) et
- un cathéter mobile, qui est pourvu d'une liaison Luer-Lock à son extrémité proximale, étant prévu dans l'aiguille de ponction à une seule lumière.

2. Dispositif selon la revendication 1, **caractérisé en ce que** l'axe de la tête à ultrasons (12) est disposé de manière à former un angle de 30° à 90° par rapport à l'axe horizontal du tube support (1).

3. Dispositif selon la revendication 1 ou 2, **caractérisé en ce que** l'élément de positionnement (4) destiné au tube de guidage (3) comporte un logement (5) en forme d'entonnoir destiné à favoriser l'introduction de l'aiguille de ponction à double lumière dans le tube de guidage (3).

4. Dispositif selon l'une au moins des revendications 1 à 3, **caractérisé en ce que** le support de tête à ultrasons (10) est relié de manière amovible au tube support (1).

5. Dispositif selon l'une au moins des revendications 1 à 4, **caractérisé en ce que** l'aiguille de ponction à double lumière est entourée d'un tube en Téflon pour augmenter la capacité de glissement dans le tube de guidage (3) et/ou le tube de guidage (3) comporte à son extrémité distale (13) un capuchon de fermeture (14) pouvant être vissé.

6. Dispositif selon l'une au moins des revendications 1 à 5, **caractérisé en ce que** le générateur d'impulsions (6) est pourvu d'une fente à son extrémité distale et le tuyau destiné au liquide de rinçage et le tuyau destiné aux ovocytes aspirés sont initialement guidés dans ladite fente (15) puis les deux tuyaux sont dirigés à travers l'alésage (18) de l'élément de fermeture (7) du tube support (1).

7. Dispositif selon l'une au moins des revendications 1 à 6, **caractérisé en ce que** la liaison amovible entre l'extrémité proximale de l'aiguille de ponction à double lumière et le générateur d'impulsions (6) est réalisée au moyen d'un manchon de raccordement (16) pouvant être vissé sur le générateur d'impulsions (6).

8. Dispositif selon l'une au moins des revendications 1 à 7, **caractérisé en ce que** des éléments de butée sont prévus au niveau du générateur d'impulsions (6) pour limiter la distance parcourue de l'aiguille de ponction à double lumière guidée en distance, un élément de butée étant prévu à l'intérieur et un élément de butée étant prévu à l'extérieur du tube support (1).

9. Dispositif selon l'une au moins des revendications 1 à 8, **caractérisé en ce qu'**un élément de verrouillage pourvu d'une fermeture à baïonnette ou une vis de fixation est prévu au niveau du générateur d'impulsions (6) pour fixer la fonction passive du dispositif et pour activer la fonction active.

10. Dispositif selon la revendication 1, **caractérisé en ce que** le tube de guidage (24) et l'auxiliaire d'introduction (28) sont conçus en deux parties et peuvent être reliés de manière amovible l'un à l'autre.

11. Dispositif selon la revendication 1 ou 10, **caractérisé en ce que** le tube de guidage (24) est revêtu d'une matière synthétique appropriée pour augmenter la capacité de glissement.

12. Dispositif selon l'une au moins des revendications 1, 10 et 11, **caractérisé en ce que** l'aiguille de ponction à une seule lumière et le cathéter sont montés de manière à pouvoir être guidés à la main et/ou une seringue jetable ou une micropipette est prévue à l'intérieur du cathéter.

13. Dispositif selon l'une au moins des revendications précédentes, **caractérisé en ce que** le dispositif est prévu et conçu pour être utilisé pour prélever des ovocytes par voie rectale ou pour transférer un embryon par voie rectale ou pour effectuer une insémination intra-utérine par voie rectale dans le rectum d'un rhinocéros, d'un éléphant ou d'un hippopotame.
